# EUROPEAN PATENT SPECIFICATION

(11) **EP 3 395 454 B1**
(45) Date of publication and mention of the grant of the patent: **02.11.2022**
(21) Application number: 17168222.2
(22) Date of filing: 26.04.2017
(51) Int. Cl.: B08B 17/06, B08B 7/00, B01F 33/841, B01F 35/10, A61L 2/10

(54) **DISPENSER FOR TINTING PASTES**
SPENDER FÜR FARBPASTEN
DISTRIBUTEUR POUR PÂTES COLORANTES

(43) Date of publication of application: 31.10.2018
(73) Proprietor: Fast & Fluid Management B.V., 2171 KZ Sassenheim (NL)
(72) Inventor: POST, Johannes Hermanus Nicolaas, 2171 NM Sassenheim (NL); BAAK, Mark, 2161 TM Lisse (NL)
(74) Representative: De Vries & Metman

(56) References cited:
- EP-A2- 1 206 976
- EP-A2- 2 266 689
- EP-B1- 1 303 347
- WO-A1-03/013748
- GB-A- 2 525 020
- US-A- 5 911 332
- US-A1- 2011 052 379
- US-A1- 2012 222 774
- US-A1- 2015 257 974
- US-B1- 6 194 530
- Xiaoxiao Sun et al: "Guanidine-based polymeric microspheres with a nonleaching, antibacterial performance", Wiley Online Library, 24 March 2017 (2017-03-24), pages 1-16, XP002774733, Retrieved from the Internet: URL:http://onlinelibrary.wiley.com/doi/10. 1002/app.44821/full [retrieved on 2017-10-17]

## Description

The present disclosure relates to a dispenser for tinting pastes. Tinting pastes are pigmented concentrates used for tinting base paints, for instance at a point of sale or a car refinish body shop. The dispensers typically comprise a plurality of canisters with a dispense nozzle, e.g., at a bottom side of the canister, and a refill opening, e.g., at a top side of the canister. The canister may comprise a pump or can selectively be connected to a pump to dispense a selected amount of the tinting paste. Optionally, the canister is provided with a stirrer. An example of a dispenser for powder colorants is disclosed in EP 2 266 689 A1. Another system that can be used for paint mixing is disclosed in US2015257974 A1, which discloses a dispenser for dispensing tinting pastes, the dispenser comprising a canister with a dispense nozzle and a refill opening and cleaning means.

Due to environmental regulations, paints and tinting pastes are usually water borne. Such aqueous compositions are sensitive for microbiological activity, in particular growth of mould, algae, bacteria or other microorganisms. This is particularly problematic for tinting pastes, which are typically stored for longer periods in canisters where only occasionally small amounts are dispensed from. Microbiological fouling and mildew can particularly occur in the head space of such canisters or at the dispensing nozzle. To prevent this, the pastes usually comprise preservatives chemically inhibiting microbiological activity. Such preservatives cause environmental and health risks.

It is an object of the invention to provide a dispenser for tinting pastes, which makes it possible to dispense tinting pastes with a reduced content of preservatives, e.g., preservative-free pastes, without increasing the risk of microbiological growth.

The object of the invention is achieved with a dispenser according to claim 1.

Particularly useful is UV-C light of a wavelength in the range of 185 - 254 nm. The UV-C light may for example be a continuous light, an intermittent light or a flash light flashing after microbiological activity has been detected or flashing at regular intervals.

Additionally, the cleaning means may for example include an anti-fouling surface contacting the tinting paste during storage or dispense, such as the inner walls of the canisters, the dispense nozzle and/or surfaces of a stirrer used in the canister. The anti-fouling surface is an ultrahydrophobic surface, e.g., to obtain a so-called lotus effect. Any condensed moisture ion the surface will directly flow down and clean the surface. A commercially available example includes the Neverwet^{®} coating available from Neverwet LLC. US patent US 9067821 and British patent application GB 2525020 disclose further examples of suitable coatings.

The anti-fouling surface can for example be formed by a coating, such as for example an anti-fouling coating comprising one or more biocidal agents. Such coatings are for example known from yacht paints industry. US 7662222 discloses suitable examples of such coatings. Inorganic coatings can also be used, such as coatings comprising combustion synthesized metallic powder, or so-called cermet, such as coating comprising ANA additives of Advanced Nano Solutions, in Dallas, United States.

Particularly suitable biocidal agents that can be used in a coating, may for example comprise micropuncturing agents or building blocks, e.g., polymeric microspheres with cationic needles, e.g., guanidine based polymeric microspheres. In a more specific embodiment, the polymeric microspheres may comprise hydrophylic blocks, e.g., of polyhexamethylene guanidine hydrochloride (PHGC) and a hydrophobic block, e.g., of poly(styrene-co-glycidyl) methacrylate (PSGMA). A suitable example of a resin comprising such agents as building blocks of the resin is MyCroFence^{®} AM 215 of Croda.

Optionally, the antifouling coating is a fouling release coating, such as a silicone coating.

Instead of a coating, or in addition to such a coating, the anti-fouling surface can be formed by a sleeve of an anti-microbiological material. Particular examples of such sleeves include form inliners available from Nittel GmbH, Raunheim, Germany.

In a further embodiment, the cleaning means may additionally comprise a dosing unit for dosing an agent neutralizing a preservative agent in the tinting paste, or in the base paint used. The neutralizing agent can for example be added at a point in a flow path of the tinting paste leaving the canister during dispensing. The dosing unit for the neutralizing agent can also be a separate unit. In such a case the usual tinting pastes and/or base paints can be used, which include one or more preservative agents. When the tinting paste is dosed and dispensed a control unit of the dispenser controls the neutralizer inlet to dose an amount of neutralizing agent into the dispensed tinting paste. As a result, the dispensed tinting paste is free of preservatives.

The control unit of the colorant dispenser can be programmed with dispense software and loaded with colorant recipes and details of amounts and characteristics of the individual colorants in relation to color and amount of preservatives. The dispense software calculates a suitable formulation based on the desired color to be dispensed and calculates the amount and type of neutralizer to be added to neutralize the active preservation components at least partly. The control unit can use time factor corrections to calculate the needed amount of neutralizer. The concentration of preservatives will decrease over time. If a recipe contains colorants A, B and C, respectively having a neutralizer content of K %, L %, and M%, the required amount Q of neutralizing agent is Q=A*K%*U+B*L%*W+C*M%*Z, with U, W, and Z being the respective time factor correction of the colorants A, B and C.

Suitable neutralizing agents include for example sodium thiosulfate which can be used for neutralizing n-butyl-1,2-benzisothizolin-3-one (BBIT). A mixture of sodium thiosulfate and sodium thioglycolic acid can be used as a neutralizing agent for the perseverative agent zinc pyrithione (ZPT) .

In a further embodiment the cleaning means may include one or more further actinic radiation sources directed to a dispense nozzle. In accordance with the invention, the cleaning means comprise at least one actinic UV source directed to the refill opening of a canister and/or the inner walls of a headspace of a canister. Such an actinic radiation source may for example be a further UV-source, such as a UV-C source, or it may be an IR source and/or a laser source. Combinations of different actinic sources are also useful. The further actinic source or sources can for example be directed to the dispense nozzle of the canister or to the refill opening or the inner walls of the head space in the canister.

In a further embodiment the cleaning means may additionally comprise an anti-microbiological filter, e.g., a HEPA filter, at a refill opening of the canister or as part of a lid covering the canister refill opening. Such filters may help to reduce the risk of contamination by microorganisms of any type.

Microbiological fouling or contamination may also be inhibited by rinsing or flushing, e.g., using a rinsing gas, such as ozone, nitrogen or steam. To that end the cleaning means comprise a gas inlet port of the canister connected or connectable to a source of the rinsing gas and a gas discharge port for discharging air from the canister.

In a further embodiment the cleaning means may for example additionally comprise temperature control means controlling the temperature in the canister, e.g., by flash pasteurization. The temperature can be increased or decreased in order to inhibit microbiological activity, e.g., at regular intervals, e.g., daily, or after detection of microbiological activity. The nozzle surface can for example be heated using infrared light, steam laser, e.g., to a temperature of at least 60°C, e.g., at least 70v, e.g. at least 72°C for at least 10 seconds, e.g., at least 15 seconds, e.g., at least 20 seconds.

Optionally, the dispenser may comprise one or more canisters with an expander within the canister and a control unit to expand the expander for volumetric compensation of a volume of dispensed tinting paste. This way, the top level of the tinting paste in the canister can be kept constant and minimal, reducing any head space volume which could give rise to microbiological activity.

Another way to reduce head space volume is to use size adjustment means for at least one of the canisters. For example, the canister may comprise a top part telescopically slideable relative to a base part, so the head space volume remains the same during dispense of tinting paste from the canister. Alternatively the canister may be shaped as a bellows or may be harmonica-shaped.

In a further embodiment the cleaning means may additionally comprise means for providing an overpressure in at least one of the canisters. This helps to prevent entrance by microorganisms and other environmental contamination. Fans can be used to provide the overpressure which may be provided with a HEPA filter, or similar microbiological filter, at their air suction sides.

The canisters may further comprise one or more spectral imaging sensors, e.g., for imaging a top surface of the tinting paste in the canister.

Suitable sensors include 4-band multi-spectral sensors for snapshoot acquisition of RGB+NIR images, such as the Avior^{®} sensors available from 3D-One NL in the Netherlands.

The imaging data can be processed and analysed to detect any shift in the properties of reflected light in the visible and/or non-visible spectra. This makes it possible to detect any microbiological activity already in a very early stage. This may for example trigger temporarily heating or cooling or exposure to UV-light or other treatment, in order to inhibit the detected microbiological activity. Spectral imaging can for example include multispectral imaging or hyperspectral imaging.

The above-described aspects will hereafter be more explained with further details and benefits with reference to the drawing showing an embodiments by way of example.
Figure 1: shows schematically a dispenser according to the invention;
Figure 2A and B: shows a canister in cross section with an expander.
Figure 3: shows a canister of adjustable length.

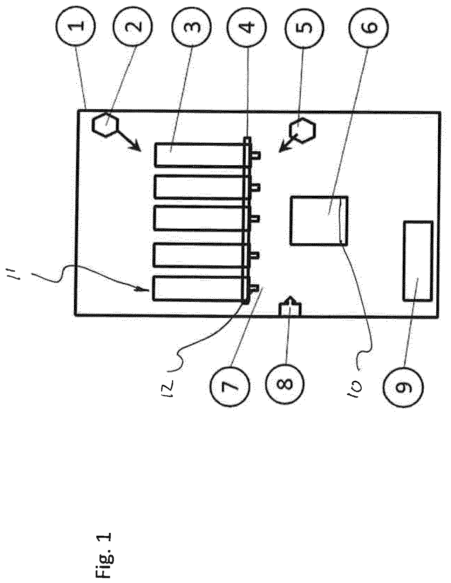

Figure 1 shows a dispenser 1 comprising a turntable 4 carrying a circular array of canisters 3, each canister 3 being filled or fillable with a tinting paste of a given colour. The dispenser 1 comprises a platform 10 for holding a paint can 6 below the canisters 3. Each canister 3 has a top end with a refill opening 11, and a bottom end with a dispensing nozzle 12. When a can is positioned on the platform 10 the turntable 4 can be activated to position a canister 3 holding a tinting paste of a selected colour above the paint can 6. The dispenser 1 may for example be provided with a user interface allowing input of a paint formulation by a user. Each canister 3 holding one of the tinting pastes used in the formulation is subsequently positioned above the paint can 6 and a pump (not shown) is driven to dispense an amount of the tinting paste into the can in accordance with the formulation. The dispenser 1 comprises a sensor 8 to detect whether or not a can 6 is present on the platform.

The dispenser 1 comprises a first UV-C source 2 at a distance above the refill openings of the canisters 3. The UV-C source 2 can be activated to flash or to radiate for a longer period in order to sterilize at least the refill opening of the nearest canister.

The dispenser 1 further comprises a second UV-C source 5 at a distance below the dispense nozzles of the canisters 3. Like the first UV-C source, also this second UV-C source 5 can be activated to flash or to radiate for a longer period in order to sterilize the dispense nozzle of the nearest canister, and optionally of further canisters. The dispenser 1 also comprises a control unit 9 for controlling the dispenser, including the UV-C sources 2, 5.

Alternatively, part 2 and/or part 5 can be a camera for checking any fouling and/or other microbiological activity within its scope.

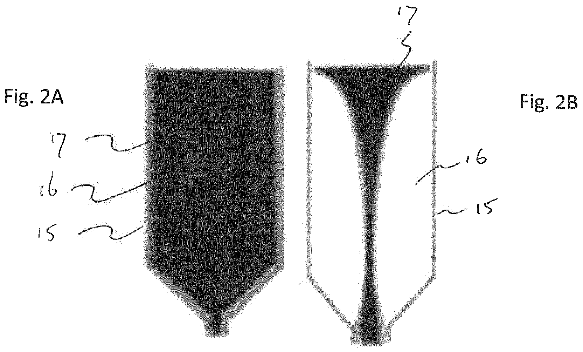

Figures 2A and 2B show a canister 15 with an expander 16, e.g., a balloon-type or bellows type expander under the control of a control unit programmed to expand the expander 16, e.g., by means of an expansion gas such as air, to compensate volumetrically for the dispensed amount of tinting paste 17. This prevents increase of the head space and reduces the risk of microbiological activity or contamination.

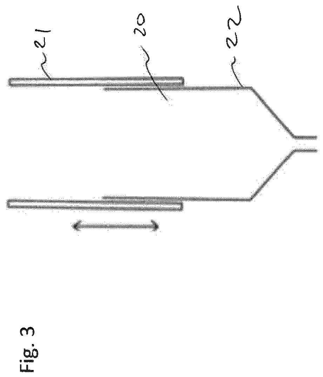

Another way of minimizing head space is to use a length adjustable canister 20, such as the one shown in Figure 3, having a top end 21 which is telescopically movable relative to a lower end 22 of the canister.

Elements and aspects discussed for or in relation with a particular embodiment may be suitably combined with elements and aspects of other embodiments, unless explicitly stated otherwise, whereby the scope of the invention is delimited by the appended claims.

## Claims

1. A dispenser (1) for selectively dispensing tinting pastes (17), the dispenser (1) comprising a plurality of canisters (3) with a dispense nozzle (12) and a refill opening (11) and cleaning means for neutralizing microorganisms and/or preservatives, wherein the cleaning means comprise at least one actinic UV-source (2) directed to the refill opening (11) of a canister (3) and/or the inner walls of a headspace of a canister (3).

2. The dispenser (1) of claim 1, wherein the neutralizing means include an anti-fouling surface contacting the tinting paste (17) during storage or dispense, such as a canister (3), a stirrer within the canister (3), a cover of the canister (3), and all further contact surfaces contacting colorant.

3. The dispenser (1) of claim 2, wherein the anti-fouling surface is an ultrahydrophobic surface.

4. The dispenser (1) of claim 2 or 3, wherein the anti-fouling surface comprises one or more biocidal agents.

5. The dispenser (1) of claim 4, wherein the one or more biocidal agents comprise micropuncturing agents, e.g., polymeric microspheres with cationic needles, e.g., polymeric microspheres comprising hydrophylic blocks, e.g., of polyhexamethylene guanidine hydrochloride and a hydrophobic block, e.g., of poly(styrene-co-glycidyl) methacrylate.

6. The dispenser (1) of claim 2, wherein the anti-fouling surface is at least partly formed by a liner or sleeve of an anti-microbiological material.

7. The dispenser (1) of any preceding claim wherein the cleaning means comprise a neutralizer inlet for dosing an agent neutralizing a preservative agent in the tinting paste in a flow path of the tinting paste (17) leaving the canister (3) during dispensing.

8. The dispenser (1) of any preceding claim, comprising at least one actinic radiation source (5) directed to a dispense nozzle (12) of a canister (3), such as a UV, e.g., UV-C source, an IR source and/or a laser source.

9. The dispenser (1) of any preceding claim wherein the cleaning means comprise an anti-microbiological filter, e.g., a HEPA filter, at a refill opening (11) of the canister (3) and/or in a lid covering the refill opening (11).

10. The dispenser (1) of any preceding claim wherein the cleaning means comprise a gas inlet port of the canister (3) connected or connectable to a source of a replacement gas, such as ozone or nitrogen, and a gas discharge port for discharging air from the canister (3).

11. The dispenser (1) of any preceding claim wherein the cleaning means comprise temperature control means controlling the temperature in the canister (3).

12. The dispenser (1) of any preceding claim comprising at least one canister (3, 15) with an expander (16) within the canister (3, 15) and control unit to expand the expander (16) for volumetric compensation of tinting paste (17) dispensed from the canister (3).

13. The dispenser (1) of any preceding claim wherein the cleaning means comprise size adjustment means for at least one of the canisters (3).

14. The dispenser (1) of any preceding claim wherein the cleaning means comprise means for providing an overpressure, e.g., in at least one of the canisters (3) or in any other space of the dispenser (1) containing colorant material.

15. A dispenser (1) for dispensing tinting pastes (17), according to any preceding claim, at least one of the canisters (3) comprising one or more spectral imaging sensors, e.g., for imaging a surface of the tinting paste (17), e.g., a tinting paste top surface in the canister (3) or a surface of the dispense nozzle (12).

## Patentansprüche

1. Spender (1) zum ausgewählten Ausgeben von Farbpasten (17), wobei der Spender (1) eine Vielzahl von Kanistern (3) mit einer Ausgabedüse (12) und einer Nachfüllöffnung (11) und Reinigungsmittel zur Neutralisierung von Mikroorganismen und/oder Konservierungsmitteln aufweist, wobei das Reinigungsmittel wenigstens eine photochemisch wirksame UV-Quelle (2) aufweist, die auf die Nachfüllöffnung (11) eines Kanisters (3) und/oder die Innenwände eines Leerraums eines Kanisters (3) ausgerichtet ist.

2. Spender (1) nach Anspruch 1, wobei das Neutralisierungsmittel eine Antifaulfläche aufweist, die mit der Farbpaste (17) während der Aufbewahrung oder dem Ausgeben in Kontakt steht, wie einen Kanister (3), ein Rührwerk im Kanister (3), eine Verkleidung des Kanisters (3) und alle weiteren Kontaktflächen, die mit dem Färbemittel in Kontakt kommen.

3. Spender (1) nach Anspruch 2, wobei die Antifaulfläche eine ultrahydrophobe Fläche ist.

4. Spender (1) nach Anspruch 2 oder 3, wobei die Antifaulfläche ein oder mehrere biozide Mittel aufweist.

5. Spender (1) nach Anspruch 4, wobei das eine oder die mehreren bioziden Mittel Mikropunktionsmittel, z. B. Polymermikrokugeln mit kationischen Nadeln, z.B. Polymermikrokugeln, die hydrophile Blöcke aufweisen, zum Beispiel Polyhexamethylenguanidinhydrochlorid und einen hydrophoben Block, z. B. aus Poly(styrol-co-glycidyl)-Methacrylat, aufweisen.

6. Spender (1) nach Anspruch 2, wobei die Antifaulfläche wenigstens teilweise durch eine Außenlage oder einer Hülse aus antimikrobiologischen Material gebildet ist.

7. Spender (1) nach einem der vorhergehenden Ansprüche, wobei das Reinigungsmittel einen Neutralisierungsmitteleinlass aufweist, um ein Mittel zu dosieren, das ein Konservierungsmittel in der Farbpaste in einem Strömungspfad der Farbpaste, die aus dem Kanister (3) heraustritt, während der Ausgabe zu neutralisieren.

8. Spender (1) nach einem der vorhergehenden Ansprüche, der wenigstens eine fotochemisch wirksame Strahlungsquelle (5) aufweist, die auf eine Ausgabedüse (12) eines Kanisters (30) ausgerichtet ist, wie eine UV, zum Beispiel UV-C-Quelle, eine IR-Quelle und/oder einer Laserquelle.

9. Spender (1) nach einem der vorhergehenden Ansprüche, wobei das Reinigungsmittel einen antimikrobiologischen Filter aufweist, zum Beispiel einen HEPA-Filter an einer Nachfüllöffnung (11) des Kanisters (3) und/oder in einer Deckelverkleidung der Nachfüllöffnung (11).

10. Spender (1) nach einem der vorhergehenden Ansprüche, wobei das Reinigungsmittel eine Gaseinlassöffnung des Kanisters (3), die mit einer Quelle eines Austauschgases, wie Ozon oder Stickstoff, verbunden oder verbindbar ist, und eine Gasauslassöffnung zum Ausgeben von Luft aus dem Kanister (3) aufweist.

11. Spender (1) nach einem der vorhergehenden Ansprüche, wobei das Reinigungsmittel Temperatursteuermittel aufweist, die die Temperatur im Kanister (3) steuern.

12. Spender (1) nach einem der vorhergehenden Ansprüche, der wenigstens einen Kanister (3, 15) mit einem Verlängerungselement (16) im Kanister (3, 15) und eine Steuereinheit zum Verlängern des Verlängerungselements (16) für eine volumenbezogene Kompensierung der aus dem Kanister ausgegebenen Farbpaste (11) aufweist.

13. Spender (1) nach einem der vorhergehenden Ansprüche, wobei das Reinigungsmittel Größenanpassungsmittel für wenigstens einen der Kanister aufweist.

14. Spender (1) nach einem der vorhergehenden Ansprüche, wobei das Reinigungsmittel Mittel zum Vorsehen eines Überdrucks aufweist, zum Beispiel in wenigstens einem der Kanister (3) oder in einem beliebigen anderen Raum des Spenders (1), der das Färbematerial enthält.

15. Spender (1) zum Ausgeben von Farbpasten (17) nach einem der vorhergehenden Ansprüche, wobei wenigstens einer der Kanister (3) ein oder mehrere spektrale Bildgebungssensoren aufweist, zum Beispiel zum Abbilden einer Fläche der Farbpaste (17), zum Beispiel eine Oberfläche der Farbpaste im Kanister (3) oder eine Fläche der Ausgabedüse (12).

## Revendications

1. Distributeur (1) pour la distribution, de manière sélective, de pâtes colorantes (17), le distributeur (1) comprenant une pluralité de réservoirs (3) ayant une buse de distribution (12) et une ouverture de réapprovisionnement (11) et des moyens de nettoyage pour la neutralisation de micro-organismes et/ou de conservateurs, dans lequel les moyens de nettoyage comprennent au moins une source (2) d'ultraviolets, UV, actiniques dirigée vers l'ouverture de réapprovisionnement (11) d'un réservoir (3) et/ou les parois internes d'un espace de tête d'un réservoir (3).

2. Distributeur (1) selon la revendication 1, dans lequel les moyens de neutralisation comportent une surface antisalissures en contact avec la pâte colorante (17) au cours d'un stockage ou d'une distribution, telle qu'un réservoir (3), un agitateur à l'intérieur du réservoir (3), un couvercle du réservoir (3) et toutes les autres surfaces de contact en contact avec un colorant.

3. Distributeur (1) selon la revendication 2, dans lequel la surface antisalissures est une surface ultrahydrophobe.

4. Distributeur (1) selon la revendication 2 ou 3, dans lequel la surface antisalissures comprend un ou plusieurs agents biocides.

5. Distributeur (1) selon la revendication 4, dans lequel les un ou plusieurs agents biocides comprennent des agents de microperforation, par ex. des microsphères polymères ayant des aiguilles cationiques, par exemple des microsphères polymères comprenant des blocs hydrophiles, par exemple, de chlorhydrate de polyhexaméthylène guanidine et un bloc hydrophobe, par exemple, de méthacrylate de poly(styrène-co-glycidyle).

6. Distributeur (1) selon la revendication 2, dans lequel la surface antisalissures est au moins partiellement formée par une doublure ou un manchon d'un matériau anti-microbiologique.

7. Distributeur (1) selon une quelconque revendication précédente dans lequel les moyens de nettoyage comprennent une entrée de neutralisant pour le dosage d'un agent neutralisant un agent conservateur dans la pâte colorante dans un chemin d'écoulement de la pâte colorante (17) quittant le réservoir (3) au cours de la distribution.

8. Distributeur (1) selon une quelconque revendication précédente, comprenant au moins une source de rayonnement actinique (5) dirigée vers une buse de distribution (12) d'un réservoir (3), telle qu'une source UV, par exemple UV-C, une source infrarouge, IR, et/ou une source laser.

9. Distributeur (1) selon une quelconque revendication précédente dans lequel les moyens de nettoyage comprennent un filtre anti-microbiologique, par exemple un filtre à haute efficacité pour les particules de l'air, HEPA, au niveau d'une ouverture de réapprovisionnement (11) du réservoir (3) et/ou dans un couvercle recouvrant l'ouverture de réapprovisionnement (11).

10. Distributeur (1) selon une quelconque revendication précédente dans lequel les moyens de nettoyage comprennent un orifice d'entrée de gaz du réservoir (3) raccordé ou apte à être raccordé à une source d'un gaz de remplacement, tel que l'ozone ou l'azote, et un orifice d'évacuation de gaz pour une évacuation de l'air du réservoir (3).

11. Distributeur (1) selon une quelconque revendication précédente dans lequel les moyens de nettoyage comprennent des moyens de régulation de température régulant la température dans le réservoir (3).

12. Distributeur (1) selon une quelconque revendication précédente comprenant au moins un réservoir (3, 15) ayant un détendeur (16) à l'intérieur du réservoir (3, 15) et une unité de commande pour détendre le détendeur (16) pour une compensation volumétrique de la pâte colorante (17) distribuée à partir du réservoir (3).

13. Distributeur (1) selon une quelconque revendication précédente dans lequel les moyens de nettoyage comprennent des moyens de réglage de dimension pour au moins l'un des réservoirs (3).

14. Distributeur (1) selon une quelconque revendication précédente dans lequel les moyens de nettoyage comprennent des moyens pour la fourniture d'une surpression, par exemple, dans au moins l'un des réservoirs (3) ou dans un quelconque autre espace du distributeur (1) contenant une matière colorante.

15. Distributeur (1) pour la distribution de pâtes colorantes (17), selon une quelconque revendication précédente, au moins l'un des réservoirs (3) comprenant un ou plusieurs capteurs d'imagerie spectrale, par exemple, pour l'imagerie d'une surface de la pâte colorante (17), par exemple, d'une surface supérieure de pâte colorante dans le réservoir (3) ou d'une surface de la buse de distribution (12).
